# EUROPEAN PATENT APPLICATION

(11) **EP 3 581 235 A1**
(43) Date of publication of application: **18.12.2019**
(21) Application number: 19180339.4
(22) Date of filing: 14.06.2019
(51) Int. Cl.: A61M 37/00, B81C 1/00

(54) **A METHOD OF PRODUCING A STRUCTURE**

(30) Priority: 14.06.2018 GB 201809783
(71) Applicant: Picofluidics Limited, St. Nicholas, Vale of Glamorgan CF5 6SH (GB)
(72) Inventor: MACNEIL, John, Cardiff, Vale of Glamorgan CF5 6SH (GB)
(74) Representative: Wynne-Jones IP Limited

(57) **Abstract**

A method of producing a structure comprising the steps of:
a) providing a substrate comprising one or more features that correspond to the shape of the structure to be produced, wherein the one or more features comprise a hydrophobic polydimethylsiloxane (PDMS) surface;
b) exposing at least a part of the hydrophobic PDMS surface to a plasma so that the part of the hydrophobic PDMS surface that is exposed to the plasma forms a hydrophilic PDMS surface;
c) depositing a seed layer onto the hydrophilic PDMS surface by electroless deposition;
d) depositing one or more metallic layers onto the seed layer by electrochemical deposition to form the structure; and
e) removing the structure from the substrate.

## Description

This invention relates to a method of producing a structure. This invention also relates to a substrate for producing a structure.

Structures having a dimension on the order of a few hundred micrometres and below (hereinafter termed "microstructures") may have many potential applications, such as microelectronic components and microneedles.

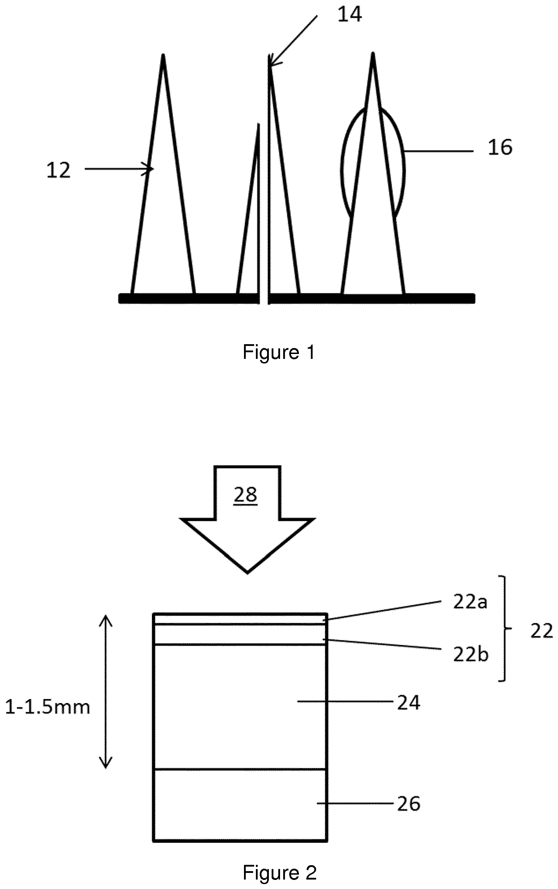

A microneedle is a needle that typically has a cross-sectional diameter of approximately 300 µm or less, and a length of approximately 1.5 mm or less. Microneedles may be solid 12 or hollow 14 structures, and can be used to deliver therapeutic substances into the skin, for example, a microneedle may be coated with a coating 16 with the substance to be delivered (Figure 1). In some instances, a microneedle may dissolve after insertion to deliver their payload. Alternatively a hollow microneedle may be used in an analogous manner to a hypodermic needle, and allows delivery and removal of fluid from a body.

Figure 2 shows a cross-section of the surface of the skin which comprises the stratum corneum 22a, the viable epidermis 22b (which together make up the epidermis 22); the dermis 24; and the subcutaneous tissue 26. Due to their size and dimensions, a microneedle 28 will typically only penetrate the skin by a distance of less than 1.5 mm. This avoids penetration into the subcutaneous tissue 26, which helps to avoid bleeding. A small microneedle diameter also helps to reduce contact with nerves in the dermis 24 thereby minimising the sensation of pain. In contrast, conventional hypodermic needles penetrate the subcutaneous tissue 26 by a few millimetres. Microneedles show great potential for the delivery of vaccines, point of care diagnostics, insulin injections and a number of other applications. However, the cost and availability of microneedle devices is still a problem after many years of development.

Microstructures, such as microneedles, are typically fabricated using photolithographic techniques and etch/deposition processes. Conventionally, a mask is applied to a substrate, and material is subsequently etched until the desired microstructures are formed. These processes have been developed for microelectronic and MEMS devices. The apparatus used in these techniques tends to be expensive to manufacture, operate and maintain. For these processes to be economically viable a very large number of devices per substrate must be fabricated or each device must have a high value. It is therefore desirable to develop an alternative, less expensive method to manufacture microstructures, such as microneedles.

As the pitch or spacing between structures increases, the number of devices per unit area decreases. Consequently, fewer devices can be fabricated per substrate and the cost is increased. Further, structures having a larger height require a greater amount of material to be etched and a thicker photoresist/mask layer. This further increases material cost, material waste and processing time. For example, longer etch steps are required to create longer microneedles.

There is a desire to develop a more economical method of manufacturing microstructures, such as microneedles. In particular, there is a desire to develop a method which reduces material cost, material waste, and decrease processing time.

The present invention in at least some of its embodiments, seeks to address some of the above described problems, desires and needs. The present invention, in at least some of its embodiments, provides a method for manufacturing or producing structures, such as microneedles.

For the avoidance of doubt, it is understood that where the terms "comprises" and "comprising" are used herein, the present specification also includes within its scope reference to the terms "includes", "including", "consists essentially of", "consisting essentially of", "consists of" and "consisting of", i.e. the terms "comprises" and "comprising" can be substituted with any of these other terms.

According to a first aspect of the invention there is a method of producing a structure comprising the steps of:
a) providing a substrate comprising one or more features that correspond to the shape of the structure to be produced, wherein the one or more features comprise a hydrophobic polydimethylsiloxane (PDMS) surface;
b) exposing at least a part of the hydrophobic PDMS surface to a plasma so that the part of the hydrophobic PDMS surface that is exposed to the plasma forms a hydrophilic PDMS surface;
c) depositing a seed layer onto the hydrophilic PDMS surface by electroless deposition;
d) depositing one or more metallic layers onto the seed layer by electrochemical deposition to form the structure; and
e) removing the structure from the substrate.

The substrate may be a template or a mould. The substrate may be a negative of the shape of the structure. The substrate may be a positive of the shape of the structure. PDMS is a long chain polymer and has been found to exhibit advantages in the production of microstructures. PDMS is a biocompatible, low cost, and naturally hydrophobic material. The structure may be removed from the flexible PDMS substrate without damaging the features of the structure, and without destroying the substrate. Advantageously, the PDMS substrate may be reused multiple times. PDMS allows micro-sized features to be formed with good resolution. Electroless deposition only occurs at parts of the substrate that have been treated so that the surface has a degraded hydrophobicity. The term hydrophilic is used here to mean a surface that is wetted by water. The hydrophilic PDMS surface has a lower contact angle towards water compared to the hydrophobic PDMS surface. Electroless deposition only occurs on parts of the substrate which are hydrophilic. The selective exposure of parts of the substrate to the plasma allows the seed layer to deposit in a desired pattern. The one or more electrochemically deposited metallic layers will only deposit on the seed layer. This allows structures to be formed having a desired pattern (e.g. hollow microneedles), and allows the features of the structure to be formed in high resolution. For example, micro-sized features may be formed with good resolution and accuracy.

The plasma may be an oxidising plasma. The plasma may be an oxygen-containing plasma. The oxygen-containing plasma may be an O₂ plasma, a clean dry air (CDA) plasma, or an O₂/Ar plasma.

Step b) may be performed at about atmospheric pressure. An atmospheric plasma avoids the need to use expensive, vacuum sealed plasma processing apparatus, for example, apparatus developed for semiconductor processing. Alternatively, a low pressure plasma may be performed at a pressure of less than 10Torr.

The method may further comprise the step of reconverting at least a part of the hydrophilic PDMS surface to the hydrophobic PDMS surface. The hydrophilic PDMS surface may be reconverted to the original hydrophobic PDMS surface by removing a surface layer of material, such as by laser ablation or by mechanical means.

The part of the hydrophobic PDMS surface that is exposed to the plasma may be defined by a mask. Using a mask helps to selectively change parts of the hydrophobic PDMS surface to the hydrophilic PDMS surface. This allows selective patterning of structure.

The seed layer may be formed from a metal, preferably copper or nickel.

The electroless deposition may be performed in the presence of a nanoparticle initiator and/or catalyst. The nanoparticle initiator and/or catalyst may comprise Sn and/or Pd nanoparticles. The seed layer may have a thickness of about 1 µm or less.

The method may further comprise the step of removing at least a part of the seed layer prior to step d). The seed layer may be removed in defined areas to avoid electrochemical deposition occurring in subsequent steps. At least a part of the seed layer may be removed by wet etching or laser ablation.

The one or more metallic layers may comprise a plurality of metallic layers. The one or more metallic layers may be formed from one or more of nickel, iron, cobalt, manganese, phosphorous, gold, silver, and/or any other noble metal. Forming the metallic layers from a plurality of metals may improve the mechanical strength of the resultant structure. The outermost metallic layer may be a noble metal, such as gold or silver. A noble metal outermost metallic layer may provide an inert surface so that the structure does not react when in contact with living tissue. The one or more metallic layers may have a total thickness of 5-150 µm, optionally 15-75 µm, optionally 10-50 µm. The thickness of the one or more metallic layers may be in a range comprising any combination of upper and lower limits provided above.

The method may further comprise the step of coating the structure with a polymer coating. The step of coating the structure with a polymer coating preferably occurs after step e). An outer polymer coating may prevent corrosion of the structure. An outer polymer coating may provide an inert surface of the structure so that metals in the structure do not react or leach when in contact with living tissue.

The substrate may be formed from monolithic PDMS. The substrate and features of the substrate may be formed simultaneously. A monolithic PDMS substrate may improve the structural integrity of the substrate.

Step e) may comprise removing the structure from the substrate non-destructively. The structure may be peeled or lifted from the substrate. The substrate may be peeled away from the structure. A non-destructive separation of the structure from the substrate allows the substrate to be reused.

The sequence of steps b) to e) may be repeated. Steps b) to e) may be repeated using the same substrate to produce a second structure. Steps b) to d) may be repeated prior to step e).

The method may further comprise the steps of:
aa) providing a mould formed from a fluoropolymer, wherein the mould comprises one or more features that correspond to the shape of the PDMS substrate;
ab) casting PDMS in the mould to form the substrate; and
ac) removing the substrate from the mould.

Step ab) may comprise thermally curing PDMS in the mould to form the substrate. PDMS may be thermally cured at a temperature of about 85-100 °C.

The fluoropolymer may be polytetrafluoroethylene (PTFE), perfluoroalkoxy (PFA), or fluorinated ethylene propylene (FEP).

Fluoropolymers provide an inert, non-stick surface for casting the PDMS substrate. This advantageously allows the mould to be used multiple times without residues forming in the features, and without the mould reacting with the PDMS. The fluoropolymer mould may be produced using a laser direct write process. The fluoropolymer mould may be a negative of the shape of the structure. The fluoropolymer mould may be a positive of the shape of the structure.

The features may be upstanding from the substrate. The features may be a negative of the shape of the structure. The features may be a positive of the shape of the structure.

The one or more features may have a cross-sectional dimension of 50-700 µm, optionally 75-500 µm, optionally 100-350 µm. The size and shape of the features are typically determined by the desired application. The features may comprise different sizes and dimensions.

The features may have a height of less than 2 mm. The features may have a height of more than 50 µm. The features may have a height of 100-1500 µm, preferably 250-1000 µm. The features may have a width or outer diameter of 50-700 µm, preferably 75-500 µm, and more preferably about 350 µm. The features may be spaced apart. The features may be spaced apart by a distance of 100-2500 µm, preferably 100-1500 µm, more preferably 500-1200 µm, and most preferably 900-1100 µm. The size, dimensions and spacing of the features may be in a range comprising any combination of upper and lower limits provided above.

The present invention may significantly reduce the amount of material required to form features of a particular size compared to known subtractive methods. This variance becomes more pronounced as the size of the features increases.

The one or more features may be tapered. Tapered features help with the removal of the structure from the substrate.

The structure may comprise a microstructure.

The structure may comprise a plurality of microneedles. A microneedle with a tapered profile aids insertion of the microneedle into tissue or other body. The microneedles may be solid or hollow microneedles. The microneedles may comprise an aperture in the tip of the microneedle or at a location along a taper.

The structure may comprise a microelectronic component, a micro-coil, an inductor, or a conductive pillar.

According to a second aspect there is a structure formed using the method according to the first aspect.

According to a third aspect there is a substrate comprising one or more features, wherein the one or more features comprise a hydrophilic polydimethylsiloxane (PDMS) surface for depositing a seed layer thereon by electroless deposition.

Whilst the invention has been described above, it extends to any inventive combination of the features set our above, or in the following description, drawings or claims. For example, any features disclosed in relation to the first aspect of the invention may be combined with any features of the second or third aspects of the invention.

Embodiments of substrates and methods in accordance with the invention will now be described with reference to the accompanying drawings, in which:
Figure 1 is a schematic illustration of a solid, hollow and coated microneedle;
Figure 2 is a schematic cross-section of the surface of the skin;
Figure 3 is a flow chart illustrating the method according to the first embodiment of the invention;
Figure 4 is a schematic cross-sectional illustration of a structure formed by the first embodiment of the invention;
Figure 5 is a schematic cross-sectional illustration of the production of a PDMS substrate from a mould;
Figure 6 is a schematic cross-sectional illustration of the formation of a seed layer on a substrate, which would lead to (A) a solid microneedle, (B) a hollow microneedle with an aperture at the tip, and (C) a hollow microneedle with an aperture at a position along the taper;
Figure 7 is a schematic cross-sectional illustration of features after an electrochemical deposition step;
Figure 8 is a conventional electrochemical deposition apparatus; and
Figure 9 is a cross-sectional view of a microneedle formed using the method of the first embodiment.

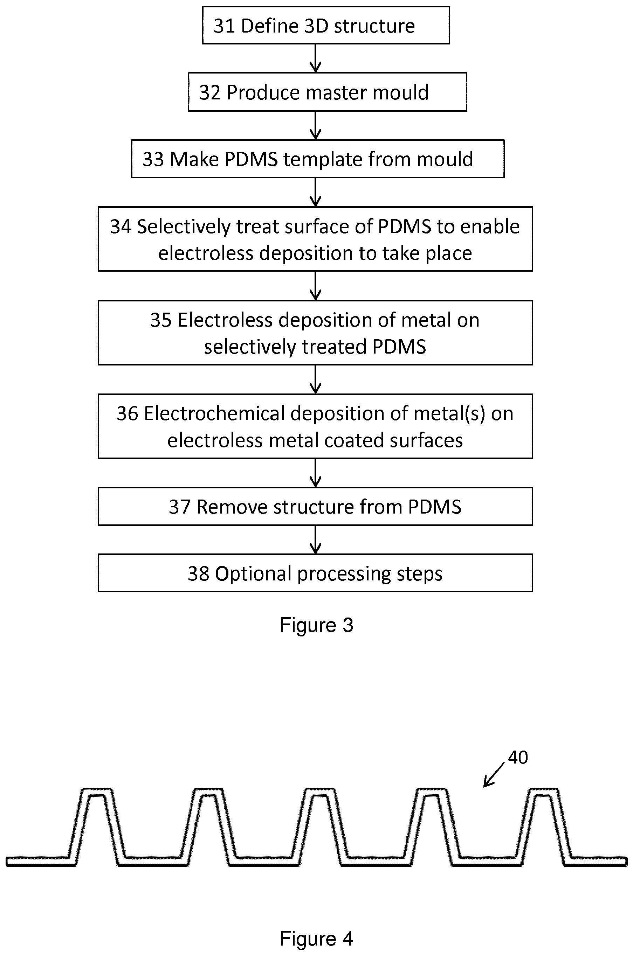

Figure 3 shows a flow chart summarising the method of manufacturing a structure according to a first embodiment of the invention. Firstly, a 3D structure is designed to be suitable for the desired end use (step 31). By way of example only, the structure may be in the form of an array of microneedles, or a microelectronic device, such as a micro-coil, an inductor or a series of conductive pillars.

In the first embodiment, the structure 40 to be formed is a 5x5 array of hollow microneedles. Figure 4 shows a simplified cross-section of a free-standing structure 40 of the first embodiment. Each microneedle has a height of 500 µm, an outer diameter (OD) of 300 µm, and a pitch of 1100 µm. Each microneedle has an internal bore with an inner diameter (ID) of 150 µm, and a wall thickness of 75 µm. The 5x5 array has a total size of 6.7 mm x 6.7 mm. It will be apparent to the skilled reader that the size and dimension of each microneedle in the array can individually be chosen to be any convenient size depending on the device design, including different sizes.

When the shape and arrangement of the structure 40 has been defined, a master mould 50 is produced (step 32). The master mould 50 has features 52 that correspond to the shape of the structure 40. The master mould may conveniently be a negative of the shape of the structure 40. Alternatively, the master mould may be a positive of the shape of the structure 40.

In the first embodiment the master mould 50 is formed from a fluoropolymer, such as polytetrafluoroethylene (PTFE), perfluoroalkoxy (PFA), or fluorinated ethylene propylene (FEP). Fluoropolymers are preferred due to their non-stick properties and due to their low chemical reactivity.

The master mould 50 is typically produced using conventional direct write laser methods. Direct write laser methods are convenient to produce a master mould including micro-scale features (i.e. a few hundred micrometres and below) with good resolution and accuracy. Despite the relatively high cost of the direct write laser process, the master mould 50 can be reused many times as required. Therefore, the direct write laser step is a single time cost.

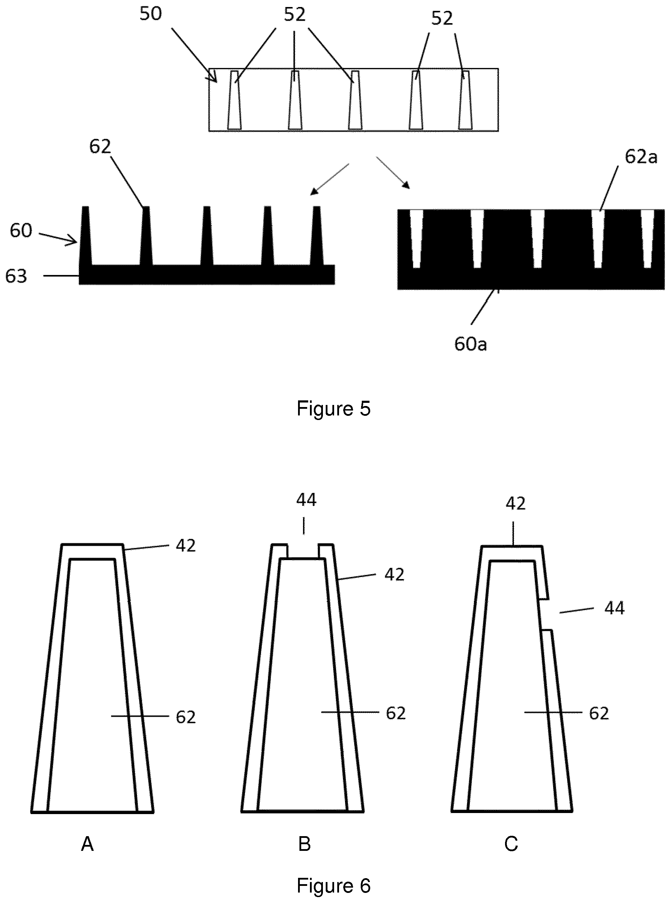

At step 33, a polydimethylsiloxane (PDMS) substrate is formed based on the master mould 50. PDMS was sourced from The Dow Chemical Company. A mixed PDMS is poured into the features 52 of the master mould 50. The PDMS is degassed and thermally cured at a temperature of 85-100 °C. The cured PDMS is subsequently removed from the master mould 50 to provide a solid, hydrophobic PDMS substrate 60, 60a (Figure 5). Since the PDMS substrate 60 is flexible and has low adhesion to the fluoropolymer mould 50, the substrate 60 can be conveniently separated from the mould 50, for example by peeling, without damaging either the substrate 60 or master mould 50. The PDMS substrate has features 62, 62a that correspond to the shape of the desired structure 40. For simplicity, only one feature 62, 62a is labelled on Figure 5. PDMS can resolve features that are as small as about 10 nm in diameter. The features 62, 62a are formed during the curing process simultaneously to the formation of the base 63 of the substrate 60. The cured PDMS substrate 60, including the base 63 and features 62, is monolithic. In the first embodiment, the PDMS substrate 60 is a positive of the shape of the structure 40. Alternatively, the PDMS substrate 60a is a negative of the shape of the structure 40.

The cured PDMS substrate 60 is a flexible and soft material having a Young's modulus of about 2 MPa. The highly flexible nature of the cured PDMS aids in the removal of the PDMS from the mast mould 50. PDMS is preferably used due to its low adhesion to the fluoropolymer master mould 50. Since PDMS does not adhere well to the fluoropolymer master mould 50, it is possible to create high aspect ratio features 62, 62a with excellent reproducibility from the master mould 50. In the first embodiment, the features 62 correspond to an array of microneedles. The microneedles have a slightly tapered tip, which aids removal of the PDMS substrate from the master mould 50, and aids penetration of the microneedles into tissue.

From a single master mould 50 it is possible to produce many PDMS substrates at a low cost and on a quick timescale. The PDMS substrate 60 is used as a substrate in subsequent processing steps.

Parts of the hydrophobic PDMS substrate 60 are subsequently subjected to a surface treatment step (step 34) to selectively reduce the hydrophobicity of these parts. In some instances, the surface treatment step 34 selectively converts the hydrophobic surface of the PDMS substrate 60 to a hydrophilic surface. Here, hydrophobic is used to mean a surface having a contact angle for water of greater than 90 °. Hydrophilic is used to mean a surface that is wetted by water.

The surface treatment step 34 typically comprises exposing parts of the PDMS substrate to an oxidising plasma. The surface treatment step 34 may be performed at a reduced pressure. Alternatively, an atmospheric plasma may be used. The plasma typically comprises oxygen. The plasma may be a clean dry air (CDA) plasma, an O₂ plasma, an O₂/Ar plasma, or any other suitable plasma. The plasma typically uses RF or DC excitation means. Without wishing to be bound by any theory or conjecture, it is believed that an oxidising plasma reacts with the surface of the PDMS. More specifically, the oxidising plasma removes methyl groups (-CH₃) from the PDMS surface to form a hydrophilic surface.

The hydrophilic surface formed by the surface treatment step 34 is suitable for use as a substrate to deposit a metallic seed layer via electroless deposition (step 35). Electroless deposition is used to deposit a metallic seed layer 42 of copper (Cu), nickel (Ni) or the like. Electroless deposition is performed using conventional methods. Typically, nanoparticle initiators, such as Pd and Sn nanoparticles, are used to initiate the electroless deposition. Other metal nanoparticle catalysts may also be used. The electroless deposition typically terminates after ∼1 µm of metal has been deposited. Electroless deposition is generally a less expensive processing technique compared to sputtering methods, such as PVD methods.

Electroless deposition only occurs on parts of the PDMS substrate that have been subjected to the surface treatment step. That is, the seed layer only forms where the PDMS is hydrophilic.

Figure 6A shows a seed layer 42 formed across the entirety of the PDMS feature 62. In this instance, the entire feature 62 was subjected to the surface treatment step 34. This configuration would lead to a solid microneedle.

In the first embodiment, the structure 40 is an array of hollow microneedles. In order to form a hollow microneedle having an aperture 44, it is desirable to avoid depositing the seed layer either at the tip of the microneedle (Figure 6B) or at a specific location along the taper (Figure 6C).

It is possible to selectively control where the electroless deposition of the seed layer occurs by selectively controlling which areas of the PDMS substrate are hydrophilic.

In one embodiment a mask layer is applied to the PDMS substrate prior to the surface treatment step 34. In this instance, only the areas that are exposed to the oxidising plasma undergo a surface change. The areas below the mask are not exposed to the plasma, and remain hydrophobic. Therefore, electroless deposition only occurs at areas that have been exposed to the surface treatment step.

In another embodiment, the PDMS substrate 60 is subjected to the surface treatment step 34 so that all of the PDMS surface becomes hydrophilic. Hydrophilic regions can be selectively converted back to their original hydrophobic state by removal of small amounts of material (∼1 µm) from the PDMS surface by, for example, laser ablation. It is noted that the change in hydrophobicity is a surface effect, and such changes occur at a depth of <<1 µm. Therefore, the selective change in hydrophobicity has a minimal influence on the shape and dimension of the PDMS substrate 60 and subsequent structure 40.

In another embodiment, the entire PDMS substrate 60 is subjected to the surface treatment step 34 so that the entire PDMS surface becomes hydrophilic. The electroless deposition step 35 forms a seed layer on the hydrophilic PDMS surface. Regions of the seed layer can subsequently be removed by wet etching or laser ablation to form a patterned seed layer 42.

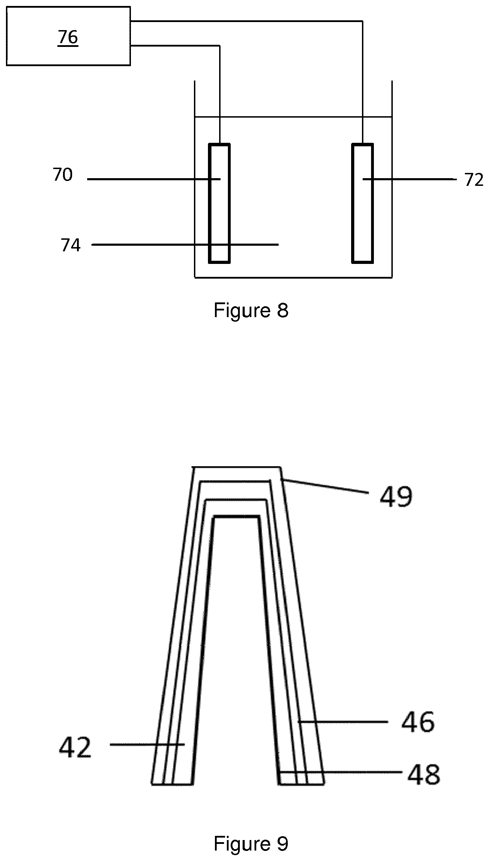

After the seed layer 42 has been deposited in the desired locations, one or more additional metal layers 46 are subsequently deposited on the seed layer 42 by electrochemical deposition (step 36). The seed layer 42 acts as a cathode and deposition surface for the electrochemical deposition process. Electrochemical deposition is performed using conventional methods. Figure 8 shows a schematic of apparatus suitable for performing the electrochemical deposition step 36. The electrochemical cell comprises an anode 70, a cathode 72 and a plating bath 74. The potential of the electrodes is controlled by a controller or power supply 76.

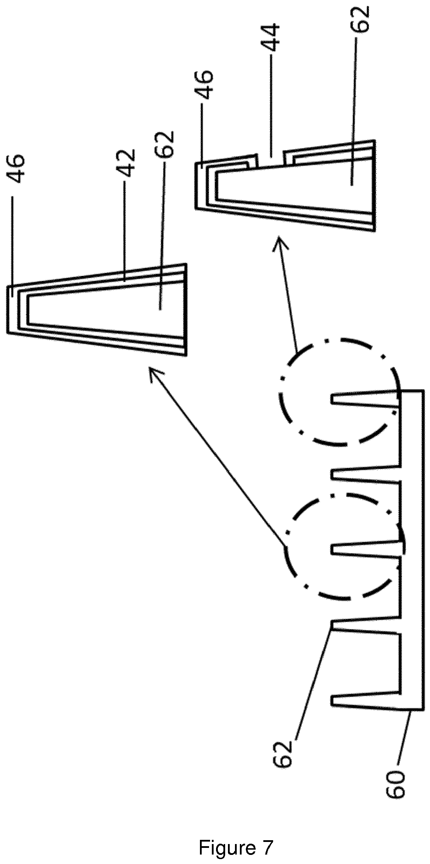

Electrochemical deposition provides a method to controllably increase the thickness of the additional metallic layers 46. The additional metallic layers 46 provide the bulk material of the structure 40. In the first embodiment, the additional metallic layers 46 correspond to the walls of the microneedle. Electrochemical deposition allows high aspect ratio features, and features having a size of several hundred micrometres and below to be produced with high resolution and good reproducibility. Electrochemical deposition does not occur in regions where the seed layer has not been formed. Electrochemical deposition therefore provides a convenient method to produce an aperture 44 in the structure, such as apertures in the tip of a microneedle (as shown in Figure 7).

The electrochemical deposition step 36 is conveniently continued or repeated until the desired metal thickness is achieved. Typically, the desired total thickness of the additional metallic layers 46 is less than about 75 µm. In some embodiments, a single metal layer 46 is formed from a single metal. In other embodiments, a plurality of metal layers 46 is formed from a plurality of metals. The additional metal layers 46 may be formed from iron, nickel, copper cobalt, manganese, phosphorous, or any other metal.

In some embodiments, it is desirable for the final external layer (i.e. the outermost layer) to be a thin layer of a noble metal, such as gold or silver. An outermost noble metal layer helps to avoid reaction if the structure 40 is used to penetrate living tissue, for example, if the structure is an array of microneedles. Such a thin layer of noble metal is conveniently deposited using conventional displacement plating techniques.

The metal (or metals) deposited via the electrochemical deposition step 36 provides the structural integrity of the resultant structure 40. Typically, depositing a plurality of metal layers formed from a plurality of metals provides a stronger structure 40.

When the desired thickness has been achieved, the resultant structure 40 is removed from the PDMS template 60. Preferably, the structure 40 is removed from the PDMS template 60 in a non-destructive manner, for example, by peeling the structure 40 away from the PDMS template 60. Since the PDMS substrate 60 is flexible, it can be separated from the structure 40 easily without damaging any features of the structure. In this way, the PDMS template 60 can be used again to make further structures. Reusing the PDMS template 60 significantly reduces the material cost and material waste during the manufacturing process.

After removal from the template, the structure 40 may be used directly or be subjected to further processing steps 38 as desired. For example, it may be convenient to coat the surface of the structure 40 that was previously in contact with the PDMS template 60. In the instance that the structure 40 is an array of hollow microneedles, the internal bore of the microneedles may be coated with a noble metal 48, such as silver. Other processing steps may also include depositing a low-friction polymer coating or laminate 49 on the outer surface of the structure 40 (Figure 9). The coatings 48, 49 act as a protective layer for the structure 40. The polymer coating 49 may prevent leaching of metal from the additional metal layers 46. The single microstructure illustrated in Figure 9 may be part of an array, such as an array of microneedles.

The resultant structure 40 is typically a microstructure, such as an array of microneedles, or a microelectronic device, such as a micro-coil, an inductor or a series of conductive pillars.

The additive process of the present invention provides an economical method to manufacture structures 40, in particular microstructures. By way of example only, a 5x5 array of microneedles was produced using the additive method of the present invention. For comparison, a 5x5 array of microneedles was produced from a substrate using a known subtractive photolithographic and etch method. In each case, the microneedles had an outer diameter (OD) of 300 µm, an inner diameter (ID) of 150 µm, and a wall thickness of 75 µm. Table 1 illustrates the normalised volumes of materials used to form the 5x5 array.

**Table 1**

| Microneedle length | Normalised subtractive vol. (comparative) | Normalised additive vol. (invention) |
|---|---|---|
| 300 µm (0.3mm) | 1 | 0.27 |
| 900 µm (0.9mm) | 3 | 0.3 |

The (comparative) subtractive photolithographic process requires at least two deep Bosch style long etch steps (e.g. DRIE etch steps) and at least two masking steps to form the microneedle array. Further etch and masking steps are required to form any additional features on the microneedles, such as a bevel. Photolithographic masking steps and plasma etch steps require expensive equipment, and are expensive processes to operate, which increases the cost of the subtractive process. The method of the present invention does not require the use of such expensive photolithographic or plasma etch apparatus. In the comparative example, ∼96% of the starting silicon substrate was etched to form the microneedle array. This leads to a significant amount of waste material. Further, when using the subtractive photolithographic process, a greater amount of the substrate must be etched if a longer microneedle is desired.

In contrast, the present invention uses an additive process to form the microneedle array. The additive approach of the present invention requires a significantly lower volume of material to form the microneedle array compared to the conventional subtractive approach. As the microneedle length increases, the variance between the subtractive approach and the present invention becomes more pronounced. The present invention is consequently particularly suited to structures comprising micro-sized features (i.e. features having a dimension of several hundred micrometres or less). For example, the present invention is particularly well-suited to produce features, such as microneedles, having a length between 100-1500 µm, an OD between 100-300 µm, and a pitch between 100-1500 µm. The method of the present invention is suitable for producing an array of features, each feature having different shapes and dimensions. Since no photolithographic processes are used in the present invention, the cost of each processing step is kept to a minimum. Additionally, after the PDMS template has been formed it can be reused multiple times. This further helps make the process of the present invention more economical.

## Claims

1. A method of producing a structure comprising the steps of:
a) providing a substrate comprising one or more features that correspond to the shape of the structure to be produced, wherein the one or more features comprise a hydrophobic polydimethylsiloxane (PDMS) surface;
b) exposing at least a part of the hydrophobic PDMS surface to a plasma so that the part of the hydrophobic PDMS surface that is exposed to the plasma forms a hydrophilic PDMS surface;
c) depositing a seed layer onto the hydrophilic PDMS surface by electroless deposition;
d) depositing one or more metallic layers onto the seed layer by electrochemical deposition to form the structure; and
e) removing the structure from the substrate.

2. The method according to claim 1 in which the plasma is an oxidising plasma.

3. The method according to claim 1 or 2 in which the plasma is an oxygen-containing plasma, such as an O₂ plasma, a clean dry air (CDA) plasma, or an O₂/Ar plasma.

4. The method according to any previous claim in which the part of the hydrophobic PDMS surface that is exposed to the plasma is defined by a mask.

5. The method according to any previous claim further comprising the step of removing at least a part of the seed layer prior to step d).

6. The method according to any previous claim in which the one or more metallic layers comprise a plurality of metallic layers.

7. The method according to any previous claim in which the one or more metallic layers are formed from one or more of nickel, iron, cobalt, manganese, phosphorous, gold, silver, and/or any other noble metal.

8. The method according to any previous claim in which step e) comprises removing the structure from the substrate non-destructively.

9. The method according to any previous claim further comprising the steps of:
aa) providing a mould formed from a fluoropolymer, such as polytetrafluoroethylene (PTFE), perfluoroalkoxy (PFA), or fluorinated ethylene propylene (FEP), wherein the mould comprises one or more features that correspond to the shape of the substrate;
ab) casting PDMS in the mould to form the substrate; and
ac) removing the substrate from the mould.

10. The method according to any previous claim in which the features are upstanding from the substrate.

11. The method according to any previous claim in which the features have a height of 50-2000 µm, preferably 100-1500 µm, and more preferably 250-1000 µm.

12. The method according to any previous claim in which the features have a width of 50-700 µm, preferably 75-500 µm, and more preferably about 350 µm.

13. The method according to any previous claim in which the one or more features are tapered.

14. The method according to any previous claim in which the structure comprises a plurality of microneedles.

15. A substrate comprising one or more features, wherein the one or more features comprise a hydrophilic polydimethylsiloxane (PDMS) surface for depositing a seed layer thereon by electroless deposition.
